# EUROPEAN PATENT APPLICATION

(11) **EP 4 834 912 A1**
(43) Date of publication of application: **04.11.2026**
(21) Application number: 24912744.0
(22) Date of filing: 23.12.2024
(51) Int. Cl.: C07H 19/048, A23L 33/13, A61K 31/706, A61P 43/00

(54) **SALT OF REDUCED BETA-NICOTINAMIDE MONONUCLEOTIDE, AND METHOD FOR PRODUCING SAME**

(30) Priority: 25.12.2023 JP 2023218492
(71) Applicant: KANEKA CORPORATION, Osaka-shi, Osaka 530-8288 (JP)
(72) Inventor: YOSHIDA, Masashi, Takasago-shi, Hyogo 676-8688 (JP); YANO, Atsushi, Takasago-shi, Hyogo 676-8688 (JP); KAWACHI, Hideo, Takasago-shi, Hyogo 676-8688 (JP); MATSUBARA, Hisanori, Takasago-shi, Hyogo 676-8688 (JP); MORIYAMA, Daisuke, Takasago-shi, Hyogo 676-8688 (JP); ITO, Noriyuki, Takasago-shi, Hyogo 676-8688 (JP)
(74) Representative: Hoffmann Eitle
(86) International application number: PCT/JP2024/045513
(87) International publication number: WO 2025/142857

(57) **Abstract**

The present disclosure aims to provide novel salts of reduced β-nicotinamide mononucleotide (NMNH), which is expected to have excellent anti-aging effects, that are easy to distribute and handle, and to provide a method for manufacturing the novel NMNH salts.

One embodiment of the present invention is a salt of reduced β-nicotinamide mononucleotide, which is at least one salt selected from the group consisting of a potassium salt, an ammonium salt, and divalent metal salts other than a calcium salt.

## Description

### Technical Field

The present disclosure relates to a salt of reduced β-nicotinamide mononucleotide and a method for manufacturing the same.

### Background Art

Nicotine adenine dinucleotide (NAD) is an essential compound for mitochondrial energy production in eukaryotes. β-nicotinamide mononucleotide (NMN) is a precursor of NAD. It has been reported that NMN supplementation can effectively increase NAD levels in vivo (see Non-Patent Literature 1).

It has also been reported that an increase in NAD levels in the body activates the sirtuin gene, which is also known as a longevity gene (see Non-Patent Literature 2), and NMN, a precursor of NAD, has attracted attention as a pharmaceutical/functional food material from the viewpoints of improving vitality and anti-aging.

Furthermore, it has been reported that reduced β-nicotinamide mononucleotide (NMNH), a reduced form of NMN, increases NAD more effectively than NMN (for example, see Non-Patent Literature 3), and NMNH is expected to be a more effective anti-aging material than NMN.

However, NMNH is unstable and presents challenges in storage and distribution. Currently, the amorphous NMNH disodium salt is commercially available on a small scale, but this disodium salt is deliquescent (see Patent Literature 1), and careful management of temperature and humidity is still required.

### Citation List

### Patent Literature

Patent Literature 1: China Patent Application Publication No. 115368423.

### Non-Patent Literature

Non-Patent Literature 1: REVOLLO, Javier R.; GRIMM, Andrew A.; IMAI, Shin-ichiro. "The NAD biosynthesis pathway mediated by nicotinamide phosphoribosyltransferase regulates Sir2 activity in mammalian cells". Journal of Biological Chemistry, 2004, 279.49: 50754-50763.
Non-Patent Literature 2: North BJ, Rosenberg MA, et al. "SIRT2 induces the checkpoint kinase BubR1 to increase lifespan". EMBO J. 2014 Jul 1;33(13):1438-53.
Non-Patent Literature 3: ZAPATA - PEREZ, Ruben, et al. "Reduced nicotinamide mononucleotide is a new and potent NAD+ precursor in mammalian cells and mice". The FASEB Journal, 2021, 35.4: e21456.

### Summary of Invention

### Technical Problem

The present disclosure aims to provide a novel NMNH salt that is easy to distribute and handle, and as well as a method for manufacturing the novel NMNH salt, which is expected to exhibit excellent anti-aging effects.

### Solution to Problem

Exemplified aspects of this embodiment are described as follows:
[1] A salt of reduced β-nicotinamide mononucleotide, wherein the salt is at least one salt selected from a potassium salt, an ammonium salt, and divalent metal salts other than a calcium salt.
[2] A salt of reduced β-nicotinamide mononucleotide according to [1], wherein the divalent metal salt is an iron salt or a magnesium salt.
[3] A method for manufacturing a salt of reduced β-nicotinamide mononucleotide, wherein the salt is at least one salt selected from a potassium salt, an ammonium salt, and divalent metal salts other than a calcium salt, comprising:
   process (I) of preparing a solution containing reduced β-nicotinamide mononucleotide and at least one ion selected from a potassium ion, an ammonium ion, and divalent metal ions other than a calcium ion in an aqueous solvent;
   process (II) of precipitating a salt of reduced β-nicotinamide mononucleotide by mixing the solution obtained in process (I) with a water-miscible organic solvent; and
   process (III) of isolating the salt precipitated in process (II).
[4] The manufacturing method according to claim 3, wherein step (I) is performed by a method comprising the following processes (A) and (B):
   process (A) of obtaining an anion-exchange resin having reduced β-nicotinamide mononucleotide adsorbed thereon by providing reduced β-nicotinamide mononucleotide to an anion-exchange resin;
   Process (B) of extracting the reduced β-nicotinamide mononucleotide in the anion-exchange resin obtained in process (A) with an aqueous solution containing at least one ion selected from a potassium ion, an ammonium ion, and divalent metal ions other than a calcium ion.
   The present specification encompasses the contents disclosed in Japanese Patent Application No. 2023-218492 on which the priority of the present application is based.

### Advantageous Effects of Invention

The present disclosure provides a novel NMNH salt and a method for manufacturing the same. The obtained NMNH salt does not exhibit deliquescent properties and is easier to distribute and handle than the conventional amorphous NMNH disodium salt.

### Description of Embodiments

The present invention will be described in detail below.

One aspect of this embodiment is a salt of reduced β-nicotinamide mononucleotide, wherein the salt is at least one salt selected from a potassium salt, an ammonium salt, and divalent metal salts other than a calcium salt. Another aspect of this embodiment is a method for manufacturing a salt of reduced β-nicotinamide mononucleotide, wherein the salt is at least one salt selected from a potassium salt, an ammonium salt, and divalent metal salts other than a calcium salt, comprising: process (I) of preparing a solution containing reduced β-nicotinamide mononucleotide and at least one ion selected from a potassium ion, an ammonium ion, and divalent metal ions other than a calcium ion in an aqueous solvent; process (II) of precipitating a salt of reduced β-nicotinamide mononucleotide by mixing the solution obtained in process (I) with a water-miscible organic solvent; and process (III) of isolating the salt precipitated in process (II). This embodiment will be described in detail below.

### (Salt of reduced β-nicotinamide mononucleotide and method for manufacturing the same)

The salt of reduced β-nicotinamide mononucleotide is at least one salt selected from a potassium salt, an ammonium salt, and divalent metal salts other than a calcium salt. Specific examples include divalent metal salts other than a calcium salt of reduced β-nicotinamide mononucleotide, a potassium salt of reduced β-nicotinamide mononucleotide, more specifically, a dipotassium salt of reduced β-nicotinamide mononucleotide, and an ammonium salt of reduced β-nicotinamide mononucleotide, more specifically, a diammonium salt of reduced β-nicotinamide mononucleotide. The salt of reduced β-nicotinamide mononucleotide has a specific cation and has improved storage stability compared to the known amorphous NMNH disodium salt.

Iron salts or magnesium salts are preferred aspects of the divalent metal salt. The ammonium cation that composes the ammonium salt is not limited to the most common NH₄⁺, but may be a primary ammonium cation, a secondary ammonium cation, a tertiary ammonium cation, or a quaternary ammonium cation. The ammonium cation can be represented, for example, by NR₄⁺ (wherein each R is independently a hydrogen atom or an organic group). Examples of the organic group include alkyl groups, aryl groups, or the like, with alkyl groups having 1 to 10 carbon atoms and aryl groups having 1 to 10 carbon atoms being preferred. NH₄⁺ is a preferred aspect of the ammonium cation.

Reduced β-nicotinamide mononucleotide is a compound represented by the following formula (1):

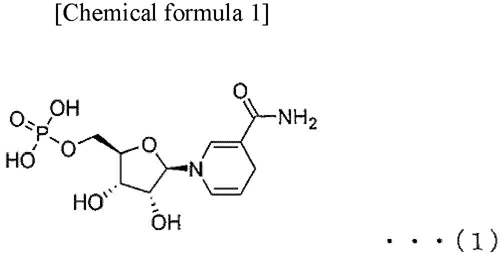

A salt of reduced β-nicotinamide mononucleotide has, for example, a structure in which two H⁺ of the phosphate group (-OP(O)(OH)₂) of the above-mentioned reduced β-nicotinamide mononucleotide are replaced by one divalent metal ion, two potassium ions, or two ammonium ions, and is, for example, a compound represented by the following formula (2), (3), or (4):

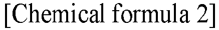

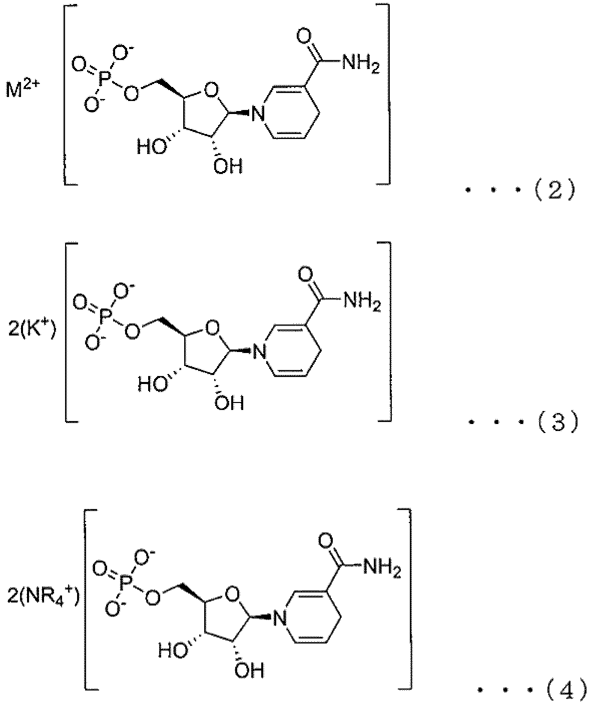

In the formula (2), M²⁺ is a divalent metal ion (divalent cation) other than a calcium ion, and may be an iron ion (Fe²⁺) or a magnesium ion (Mg²⁺). In the formula (4), R is each independently a hydrogen atom or an organic group. Examples of the organic group include alkyl groups, aryl groups, and the like, and alkyl groups having 1 to 10 carbon atoms and aryl groups having 1 to 10 carbon atoms are preferred. One preferred aspect is that all R are hydrogen atoms.

There are no particular limitations on the production conditions for obtaining each NMNH salt, and they may be produced by, for example, the method described in the examples. Examples of methods for manufacturing NMNH salts include process (I) of preparing a solution containing reduced β-nicotinamide mononucleotide and at least one ion selected from a potassium ion, an ammonium ion, and divalent metal ions other than a calcium ion in an aqueous solvent; process (II) of precipitating a salt of reduced β-nicotinamide mononucleotide by mixing the solution obtained in process (I) with a water-miscible organic solvent; and process (III) of isolating the salt precipitated in process (II).

As the aqueous solvent, water is the preferred aspect. In process (I), reduced β-nicotinamide mononucleotide or the disodium salt of reduced β-nicotinamide mononucleotide may be added to the aqueous solvent.

Furthermore, to supply at least one ion selected from divalent metal ions other than a calcium ion, a potassium ion, and an ammonium ion, at least one compound or salt selected from divalent metal (other than calcium) compounds or salts, potassium compounds or salts, and ammonium compounds or salts may be added. These compounds or salts may be used alone or in combination of two or more. When these compounds or salts are added to an aqueous solvent, they may be solid or aqueous solutions of these compounds or salts. Examples of divalent metal (other than calcium) compounds or salts, potassium compounds or salts, and ammonium compounds or salts include, but are not limited to, iron(II) chloride, magnesium chloride, potassium chloride, and ammonium chloride. Hydrates of these compounds or salts may also be used.

Process (I) may be carried out by a method comprising the following processes (A) and (B).
process (A) of obtaining an anion-exchange resin having reduced β-nicotinamide mononucleotide adsorbed thereon by providing reduced β-nicotinamide mononucleotide to an anion-exchange resin;
Process (B) of extracting the reduced β-nicotinamide mononucleotide in the anion-exchange resin obtained in process (A) with an aqueous solution containing at least one ion selected from a potassium ion, an ammonium ion, and divalent metal ions other than a calcium ion.

A method comprising processes (A) and (B) is preferred because it can eliminate cations other than those constituting the desired salt of reduced β-nicotinamide mononucleotide (cations other than the target).

The temperature at which process (I) is carried out is not particularly limited as long as NMNH does not decompose, but is usually 0 to 40°C, preferably 0 to 30°C.

The pH of the solution obtained in process (I) is not particularly limited as long as it is within a range in which NMNH does not decompose, but is usually pH 8 to 11, preferably pH 9 to 10. When adjusting the pH, sodium hydroxide or the like, or an organic acid or the like may be used as appropriate.

The concentrations of NMNH and the ions contained in the solution decrease over time due to precipitation of NMNH salts. Immediately after preparation of the solution, the concentrations of NMNH and the ions are each independently preferably 20 mM or more, more preferably 80 mM or more. The concentrations of NMNH and the ions may be each independently at or below a concentration at which they can be dissolved in a solvent, and are preferably 1.5 M or less, more preferably 450 mM or less.

In process (II), the solution obtained in process (I) is mixed with a water-miscible organic solvent to precipitate the salt of reduced β-nicotinamide mononucleotide. The water-miscible organic solvent is preferably an organic solvent that is miscible with water, but functions as a poor solvent for the salt of reduced β-nicotinamide mononucleotide, and at least one solvent selected from, for example, methanol, ethanol, propanol, 2-propanol, acetonitrile, hexane, acetone, and DMSO may be used.

Process (II) is usually carried out under stirring. The temperature during process (II) is not particularly limited, but is usually 0 to 40°C, preferably 0 to 30°C. The time during which process (II) is carried out is not particularly limited, but is usually more than 0 hours and 10 hours or less, preferably 1 to 5 hours.

Process (III) is a process for isolating the salt precipitated in process (II), which may be performed by, for example, filtration, removal of the supernatant, or the like. The isolated salt (NMNH salt) may be washed with an alcohol or the like.

The isolated salt (salt of NMNH) may be subjected to a drying process, such as atmospheric-pressure drying, reduced-pressure drying, or the like.

### (Uses, Compositions)

The salt of reduced β-nicotinamide mononucleotide described above does not exhibit deliquescent properties, and thus is easier to distribute and handle than the conventional amorphous NMNH disodium salt, and can be used in a variety of applications in which NMNH is used. For example, the salt of reduced β-nicotinamide mononucleotide or a composition comprising the salt of reduced β-nicotinamide mononucleotide may be used as a food, a nutrient- functional food, a food for specified health uses, a nutritional supplement, a beverage, a cosmetic, a pet food, an animal drug, a pharmaceutical, a therapeutic drug, or a preventive drug. When used for these purposes, the composition is usually a composition comprising the salt of reduced β-nicotinamide mononucleotide and at least one component selected from ingredients acceptable for the intended use, such as excipients, disintegrants, lubricants, binders, antioxidants, colorants, anti-aggregating agents, absorption enhancers, solubilizers, stabilizers, viscosity modifiers, oils and fats, surfactants, and active ingredients other than salts of reduced β-nicotinamide mononucleotide.

### Examples

The present embodiment will be explained below using examples, but the present disclosure is not limited to these examples.

The measurement conditions for high-performance liquid chromatography (HPLC), ion chromatography, and iron ion concentration used in the Examples are shown below.

### (HPLC Measurement Conditions)

Apparatus: Shimadzu SIL20 series
Column: COSMOSIL 3PBr Packed Column, 3.0 mm I.D. (inner diameter) x 150 mm
Mobile phase: 20 mM ammonium formate/methanol = 95/5 (v/v)
Flow rate: 0.4 mL/min
Detection wavelength: 340 nm or 260 nm

### (Ion Chromatography Measurement Conditions)

Apparatus: Thermo Scientific Dionex Integration HPIC
Column: Dionex IonPac CS16
Mobile phase: 30 mM methanesulfonic acid
Flow rate: 1 mL/min

### (Iron Ion Concentration Measurement Conditions)

Detection method: Colorimetric determination using Roche IRON BIO (This detection method comprises the following three steps, and detection is performed at an absorbance of 552 nm: (1) liberation of iron ions in the sample under acidic conditions; (2) reduction of iron(III) to iron(II) by ascorbic acid; and (3) formation of a complex between FerroZine and iron(II)).
Detection wavelength: 552 nm

### [Example 1]

### (Preparation of Iron Salt of Reduced β-Nicotinamide Mononucleotide)

1.0043 g (2.55 mmol) of a disodium salt of reduced β-nicotinamide mononucleotide (NMNH) and 0.3803 g (3.00 mmol) of iron(II) chloride were dissolved in 10 mL of water under a nitrogen atmosphere. The resulting solution was stirred at room temperature to induce precipitation, and 10 mL of ethanol was added, resulting in the formation of an increased amount of precipitate. The precipitate was filtered, washed with 20 mL of 50% ethanol and 20 mL of 100% ethanol, and then dried under reduced pressure to obtain an iron salt of reduced β-nicotinamide mononucleotide.

10.3 mg of a reduced β-nicotinamide mononucleotide iron salt was dissolved in 10 mL of a 20 mM EDTA aqueous solution to prepare an aqueous solution of an NMNH salt. The NMNH content of the aqueous solution of an NMNH salt was measured using HPLC with a detection wavelength set to 340 nm, yielding a content of 26.0% (w/w). The NMN content was measured using HPLC with a detection wavelength set to 260 nm, yielding a content of 12.6% (w/w). Furthermore, the aqueous solution of the NMNH salt was analyzed using IRON BIO, and the iron content was measured, yielding a content of 8.0% (w/w). From these contents, the content ratio of NMNH, NMN, and iron was calculated, resulting in NMNH, NMN, and iron being contained in a molar ratio of 1.00 : 0.48 : 1.92. The high ratio of iron to NMNH is attributed to the oxidation of some of the NMNH during handling in air.

### [Example 2]

### (Preparation of Magnesium Salt of Reduced β-Nicotinamide Mononucleotide)

0.2003 g (0.50 mmol) of the disodium salt of reduced β-nicotinamide mononucleotide (NMNH) and 0.1546 g (0.76 mmol) of magnesium chloride hexahydrate were dissolved in 10 mL of water. 15 mL of ethanol was added to the resulting solution, and the mixture was stirred at room temperature overnight to precipitate. The precipitate was filtered, washed with 20 mL of 75% ethanol and 20 mL of 100% ethanol, and then dried under reduced pressure to obtain the magnesium salt of reduced β-nicotinamide mononucleotide.

8.0 mg of the magnesium salt of reduced β-nicotinamide mononucleotide was dissolved in 20 mL of water to prepare an aqueous solution of an NMNH salt. The NMNH content of the aqueous solution of an NMNH salt was measured using HPLC with a detection wavelength of 340 nm, yielding a content of 79.0% (w/w). Furthermore, the aqueous solution of an NMNH salt was analyzed by ion chromatography to measure the magnesium content, yielding a content of 4.3% (w/w). Based on these contents, the content ratio of NMNH to magnesium was calculated, resulting in a molar ratio of NMNH to magnesium of 1.00:0.76. A small amount of sodium was detected in the obtained salt, and in view of the molar ratio of NMNH to magnesium, it was considered that some sodium salt was also present.

### [Example 3]

### (Preparation of Magnesium Salt of Reduced β-Nicotinamide Mononucleotide)

5.0043 g of the disodium salt of reduced β-nicotinamide mononucleotide (NMNH) was weighed and dissolved in 50 mL of water, and 18 mL of this solution was subjected to 5.9393 g of anion-exchange resin. After stirring for 1 hour, the resin was washed three times with water, and 20 mL of 1 M magnesium chloride was added and stirred for 1 hour. Thereafter, 80 mL of ethanol was added to the resulting supernatant and stirred overnight. The resulting precipitate was washed with 20 mL of 80 % ethanol and 20 mL of 100% ethanol, and then dried under reduced pressure to obtain a magnesium salt of reduced β-nicotinamide mononucleotide.

10.1 mg of the magnesium salt of reduced β-nicotinamide mononucleotide was dissolved in 20 mL of water to prepare an aqueous solution of an NMNH salt. The NMNH content of the aqueous solution of an NMNH salt was measured using HPLC with a detection wavelength of 340 nm, yielding a content of 69.9% (w/w). Furthermore, the aqueous solution of an NMNH salt was analyzed by ion chromatography to measure the magnesium content, yielding a content of 4.8% (w/w). Based on these contents, the content ratio of NMNH to magnesium was calculated, yielding a molar ratio of NMNH to magnesium of 1.00 : 0.95.

### [Example 4]

### (Preparation of Dipotassium Salt of Reduced β-Nicotinamide Mononucleotide)

5.0043 g of the disodium salt of reduced β-nicotinamide mononucleotide (NMNH) was weighed and dissolved in 50 mL of water, and then 20 mL of the solution was removed and 0.2361 g of potassium chloride was added to the solution. After stirring, 80 mL of methanol was added, and the mixture was stirred overnight. The resulting precipitate was washed with 80% methanol and 100% methanol, and then dried under reduced pressure to obtain a dipotassium salt of reduced β-nicotinamide mononucleotide.

10.9 mg of the dipotassium salt of reduced β-nicotinamide mononucleotide was dissolved in 20 mL of water to prepare an aqueous solution of an NMNH salt. The NMNH content of the aqueous solution of an NMNH salt was measured using HPLC with a detection wavelength of 340 nm, yielding a content of 61.9% (w/w). Furthermore, the aqueous solution of an NMNH salt was analyzed by ion chromatography, and the potassium content was measured, yielding a content of 18.3% (w/w). Based on these contents, the content ratio of NMNH to potassium was calculated, yielding a molar ratio of NMNH to potassium of 1.00 : 2.55.

### [Example 5]

### (Preparation of Diammonium Salt of Reduced β-Nicotinamide Mononucleotide)

3.5130 g of the disodium salt of reduced β-nicotinamide mononucleotide (NMNH) was weighed and dissolved in 35 mL of water, and 10 mL of this solution was subjected to 3.0098 g of anion-exchange resin. After 4 hours of stirring, the resin was washed three times with water, and 10 mL of a 1 M aqueous ammonium chloride solution was added and stirred for 2 hours. Thereafter, 60 mL of ethanol was added to the resulting supernatant and stirred overnight. The supernatant was removed and the mixture was dried under reduced pressure to obtain a diammonium salt of reduced β-nicotinamide mononucleotide.

19.8 mg of the diammonium salt of reduced β-nicotinamide mononucleotide was dissolved in 20 mL of water to prepare an aqueous solution of NMNH salt. The NMNH content of the aqueous solution of NMNH salt was measured using HPLC with a detection wavelength of 340 nm, yielding a content of 58.1% (w/w). Furthermore, the aqueous solution of NMNH salt was analyzed by ion chromatography, and the ammonium (NH₄⁺) content was measured, yielding a content of 8.1% (w/w). From these contents, the content ratio of NMNH to ammonium was calculated, yielding a molar ratio of 1.00: 2.59.

### [Example 6]

### (Deliquescence Test)

Each powder was left to stand in a thermo-hygrostat at 25°C and 60% humidity for 24 hours, and then the shape was visually confirmed. It was found that the particles of NMNH disodium salt adhered to one another and lost powder fluidity, whereas all NMNH salts of Examples 1 to 5 maintained their powder fluidity.

Each upper-limit value and/or lower-limit value of the numeric ranges described in the present specification can be arbitrarily combined to define a preferred range. For example, the upper- and lower-limit values of a numeric range may be arbitrarily combined to define a preferred range; the upper-limit values of a numeric range may be arbitrarily combined to define a preferred range; and the lower-limit values of a numeric range may be arbitrarily combined to define a preferred range. Furthermore, in the present application, a numeric range expressed using the term "to" comprises the numerical values described before and after the term "to" as the upper and lower limits, respectively.

Although the present embodiment has been described in detail above, the specific configuration is not limited to this embodiment, and various modifications and design changes without departing from the spirit and scope of the present disclosure, they are also encompassed by the present disclosure.

All publications, patents, and patent applications cited in the description are incorporated in their entirety by reference.

## Claims

1. A salt of reduced β-nicotinamide mononucleotide, wherein the salt is at least one salt selected from a potassium salt, an ammonium salt, and divalent metal salts other than a calcium salt.

2. A salt of reduced β-nicotinamide mononucleotide according to claim 1, wherein the divalent metal salt is an iron salt or a magnesium salt.

3. A method for manufacturing a salt of reduced β-nicotinamide mononucleotide, wherein the salt is at least one salt selected from a potassium salt, an ammonium salt, and divalent metal salts other than a calcium salt, comprising:
process (I) of preparing a solution containing reduced β-nicotinamide mononucleotide and at least one ion selected from a potassium ion, an ammonium ion, and divalent metal ions other than a calcium ion in an aqueous solvent;
process (II) of precipitating a salt of reduced β-nicotinamide mononucleotide by mixing the solution obtained in process (I) with a water-miscible organic solvent; and
process (III) of isolating the salt precipitated in process (II).

4. The manufacturing method according to claim 3, wherein step (I) is performed by a method comprising the following processes (A) and (B):
process (A) of obtaining an anion-exchange resin having reduced β-nicotinamide mononucleotide adsorbed thereon by providing reduced β-nicotinamide mononucleotide to an anion-exchange resin;
Process (B) of extracting the reduced β-nicotinamide mononucleotide in the anion-exchange resin obtained in process (A) with an aqueous solution containing at least one ion selected from a potassium ion, an ammonium ion, and divalent metal ions other than a calcium ion.
